# EUROPÄISCHE PATENTSCHRIFT

(11) **EP 4 376 728 B1**
(45) Veröffentlichungstag und Bekanntmachung des Hinweises auf die Patenterteilung: **05.03.2025**
(21) Anmeldenummer: 22757533.9
(22) Anmeldetag: 25.07.2022
(51) Int. Cl.: A61B 17/29, A61B 34/00, A61B 17/00

(54) **CHIRURGISCHES INSTRUMENT UND LENKGETRIEBE DAFÜR**
SURGICAL INSTRUMENT AND STEERING GEAR FOR SAME
INSTRUMENT CHIRURGICAL ET DISPOSITIF DE DIRECTION ASSOCIÉ

(30) Priorität: 28.07.2021 DE 102021119522
(43) Veröffentlichungstag der Anmeldung: 05.06.2024
(73) Patentinhaber: Karl Storz SE & Co. KG, 78532 Tuttlingen (DE)
(72) Erfinder: SCHNEIDER, Janosz, 78532 Tuttlingen (DE); LÄNGLE, Dominik, 78532 Tuttlingen (DE); GRÜNER, Sven Axel, 78532 Tuttlingen (DE); STEFAN, Jochen, 78532 Tuttlingen (DE)
(86) Internationale Anmeldenummer: PCT/EP2022/070776
(87) Internationale Veröffentlichungsnummer: WO 2023/006655

(56) Entgegenhaltungen:
- DE-A1- 102019 121 092
- US-A1- 2010 228 284
- US-A1- 2016 066 982

## Beschreibung

Die Erfindung betrifft ein chirurgisches Instrument und ein Lenkgetriebe dafür.

Aus dem Stand der Technik sind chirurgische Instrumente bekannt, die manuell oder von einem Roboter geführt werden können und die Werkzeuge aufweisen, deren Werkzeugspitze mittels mehrerer ineinandergreifender Schwenkglieder verschwenkt werden kann. Diese Schwenkglieder sind mit einer Vielzahl Lenkdrähte oder -seile verbunden, um eine feinfühlige Steuerung der Werkzeugspitze zu erreichen. Mit vielen dünnen Lenkdrähten gegenüber wenigen dickeren Lenkdrähten kann eine gleichmäßigere Kraftverteilung in alle Abwinkelungsrichtungen erzielt werden.

Ein gattungsgemäßes chirurgisches Instrument ist bspw. aus der US 5 454 827 bekannt, bei dem die distalseitigen Schwenkglieder über vier Lenkdrähte mit einer proximalseitig angeordneten räumlich verstellbaren Taumelscheibe so gekoppelt sind, dass eine Bewegung der räumlich verstellbaren Taumelscheibe eine entsprechende relative Bewegung der distalseitigen Schwenkglieder und somit ein Verschwenken der Werkzeugspitze verursacht, wobei das Bewegen der räumlich verstellbaren Taumelscheibe manuell über eine Art Joystick erfolgt, der direkt mit dieser gekoppelt ist.

Die Ausbildung des Antriebs für die Lenkdrähte mit der räumlich verstellbaren Taumelscheibe, an der alle vier Lenkdrähte gelagert sind, hat den Vorteil, dass dies eine räumlich kompakte Bauweise ermöglicht und nur ein Bauteil bewegt werden muss, um alle Lenkdrähte ansprechen zu können.

Nachteilig an dieser bekannten Konstruktion ist die Verwendung einer nur geringen Anzahl von Lenkdrähten, nämlich von nur vier Lenkdrähten, und ferner die ausschließlich manuelle Betätigbarkeit der als Antrieb für die Lenkdrähte dienenden räumlich verstellbaren Scheibe, wodurch eine feinfühlige und reproduzierbare Verstellung der distalseitigen Schwenkglieder kaum möglich ist.

In der US 7 699 855 ist ein chirurgisches Instrument offenbart, das eine Schnittstelle aufweist, um das Instrument mit einem Roboter-Arm verbinden zu können. Dabei sind alle Antriebe, die das Instrument steuern, in dem Roboter-Arm angeordnet. Die Übertragung der Drehwinkel von Antrieben zum Instrument erfolgt über Kupplungsscheiben in einer gemeinsamen Trenn-Ebene, sodass die Antriebsachsen parallel zueinander und senkrecht zu dieser Ebene stehen.

Die WO 2014 / 004 242 beschreibt ebenfalls eine solche Schnittstelle, wobei auch hier die achsparallelen Antriebe in dem Roboter-Arm verbaut sind.

Die vorstehende Gestaltung ist verbunden mit einem komplexen Aufbau und einer indirekten und spielbehafteten Ansteuerung, weil die Antriebe nicht in dem chirurgischen Instrument direkt angeordnet sind. Das daraus resultierende nicht lineare Übertragungsverhalten der Antriebsbewegung auf die Taumelscheibe kann zudem nur schlecht in einer Software-Steuerung abgebildet werden.

Auch US 10,105,128 B2 offenbart eine Ansteuerung einer solchen Werkzeugspitze; dort erfolgt dies über eine Mechanik, die Zahnscheiben-Segmente und Gelenkstangen umfasst, um die Bewegung der Antriebe auf die Taumelscheibe zu übertragen.

Die DE 10 2019 121 092 A1 beschreibt ein medizinisches Instrument mit einem hohlen Schaft, an dessen distalen Ende ein angeordnetes Instrument mittels eines Gelenkmechanismus relativ zur Längsachse des Schaftes schwenkbar ist, wobei der Gelenkmechanismus über in Längsrichtung des Schafts verlaufende Lenkdrähte mit einem proximalen Antrieb verbunden ist, wobei die Lenkdrähte an einer räumlich verstellbaren Scheibe des proximalen Antriebs gelagert sind und der motorisierte Antrieb aus zwei um 180° zueinander versetzt angeordneten Antriebseinheiten besteht, deren Antriebswellen auf einer gemeinsamen Mittelachse liegen, und die räumlich verstellbare Scheibe zwischen den zwei Antriebseinheiten angeordnet ist. Die Antriebseinheiten sind jeweils als über Motoren angetriebene Zahnräder ausgebildet, wobei jedes der beiden Zahnräder als einfaches Antriebsrad mit nur einem Zahnradkranz ausgebildet ist. Bevorzugt greifen die beiden angetriebenen Zahnräder der Antriebseinheiten in ein drittes Zahnrad ein, welches mit der räumlich verstellbaren Scheibe gekoppelt ist.

Ausgehend von diesem Stand der Technik ist es Aufgabe der vorliegenden Erfindung, ein verbessertes Lenkgetriebe für ein chirurgisches Instrument bereitzustellen, das einen Antrieb der räumlich verstellbaren Taumelscheibe mit linearem Übertragungsverhalten hat. Diese Aufgabe wird durch ein Lenkgetriebe mit den Merkmalen des Anspruchs 1 gelöst. Die weitere Aufgabe ein chirurgisches Instrument bereitzustellen, dessen räumlich verstellbare Taumelscheibe durch ein konstruktiv einfaches und platzsparendes Lenkgetriebe angetrieben wird, wird durch das chirurgische Instrument mit den Merkmalen des abhängigen Anspruchs 8 gelöst.

Weiterbildungen und bevorzugte Ausführungsformen des Lenkgetriebes und des chirurgischen Instruments sind in den Unteransprüchen ausgeführt.

Gemäß einer ersten Ausführungsform des erfindungsgemäßen Lenkgetriebes für ein chirurgisches Instrument, wobei das Lenkgetriebe am proximalen Ende eines hohlen Schafts anordenbar ist, der eine Längsachse B des Instruments definiert und am distalen Ende eine Abwinkelungsmechanik des chirurgischen Instruments aufweist, verfügt dieses über zwei motorisierte Antriebe und ist dazu ausgebildet, über die Stellwinkel der zwei Antriebe eine Taumelscheibe räumlich auszurichten. Diese wiederum ist dazu ausgebildet, die distale Abwinkelungsmechanik des chirurgischen Instruments zu steuern.

Erfindungsgemäß weist der erste Antrieb ein von einem ersten Motor angetriebenes erstes Antriebsrad auf, das als Doppelrad mit einem ersten Kegelradkranz und einem ersten Antriebskranz ausgebildet ist. Auch der zweite Antrieb weist ein von einem zweiten Motor angetriebenes zweites Antriebsrad auf, das als Doppelrad mit einem zweiten Kegelradkranz und einem zweiten Antriebskranz ausgebildet ist. Die Taumelscheibe ist zwischen den zwei Antriebsrädern, die eine gemeinsame Drehachse A aufweisen, angeordnet, wobei die Kegelradkränze einander zugewandt sind. Die Antriebskränze beider Doppelräder stehen mit jeweils einem Ritzel in Wirkverbindung, wobei ein erstes Ritzel, das mit dem ersten Antriebskranz in Wirkverbindung steht, über eine erste Antriebswelle, die eine erste Antriebsachse C definiert, durch den ersten Motor antreibbar ist. Ein zweites Ritzel, das mit dem zweiten Antriebskranz in Wirkverbindung steht, ist über eine zweite Antriebswelle, die eine zweite Antriebsachse C' definiert, durch den zweiten Motor antreibbar. Dabei verlaufen die zwei Antriebsachsen C, C' parallel zueinander und parallel zu der gemeinsamen Drehachse A der zwei Antriebsräder, sodass die Antriebsachsen bauraumsparend nahe an der Längsachse B des Instruments positioniert werden können.

Durch den motorisierten Antrieb der räumlich verstellbaren Scheibe über das Lenkgetriebe, das mit den angetriebenen Doppelräder ein lineares Übertragungsverhalten aufweist, ist es möglich, die distalseitige Werkzeugspitze exakt, feinfühlig in kleinsten Schritten und auch reproduzierbar zu aktivieren. Die Doppelräder können dabei wahlweise einstückig gefertigt sein, aber es ist auch möglich, dass die Doppelräder jeweils aus einem Kegelrad und einem Antriebsrad, die miteinander verbunden sind, bestehen. Aus der Kombination von Antriebsradkranz und Kegelradkranz wird eine direkte Übertragung der durch die Antriebe initiierten Bewegungen auf die Taumelscheibe erreicht.

In einer Ausführungsform des erfindungsgemäßen Lenkgetriebes können die zwei Doppelräder derart in Bezug auf die Taumelscheibe angeordnet sein, dass die zwei Antriebsachsen C, C' und die gemeinsame Drehachse A der zwei Doppelräder senkrecht, d. h. im rechten Winkel zu der Längsachse B des chirurgischen Instruments, verlaufen. Platzsparend können hierbei beide Motoren nebeneinander auf einer Seite in Bezug auf die Längsachse B senkrecht dazu angeordnet werden, sodass die Bauhöhe reduziert werden kann. Je nach vorhandenem Bauraum ist aber auch eine Anordnung der beiden Motoren diametral versetzt zueinander in Bezug auf die Längsachse B denkbar.

In einer dazu alternativen Ausführungsform des erfindungsgemäßen Lenkgetriebes können die beiden Antriebsräder als Hohl-Doppelräder ausgebildet sein und derart in Bezug auf die Taumelscheibe angeordnet werden, dass die gemeinsame Drehachse A der zwei Hohl-Doppelräder mit der Längsachse B des chirurgischen Instruments fluchtet bzw. zusammenfällt, und die zwei Antriebsachsen C, C' parallel zu der Längsachse B des chirurgischen Instruments verlaufen. Auch hierdurch ist eine bauraumsparende Anordnung der Motoren in der Nähe der Längsachse - und zwar parallel dazu - ermöglicht.

In beiden Anordnungsvarianten des Lenkgetriebes können die Antriebskränze der Doppelräder bzw. Hohl-Doppelräder Zahnkränze und die Ritzel entsprechend Zahnräder sein, die mit den als Zahnkränzen ausgebildeten Antriebskränzen direkt in Eingriff stehen bzw. kämmen.

Als Alternative zu der Wirkverbindung aus Zahnkranz und Zahnrad sieht eine weitere Ausführungsform vor, dass die Antriebskränze der Doppelräder bzw. Hohl-Doppelräder und die Ritzel jeweils als Riemenscheiben mit einem Umfangsprofil für einen Antriebsriemen ausgebildet sind, wobei ein erster Antriebsriemen die Wirkverbindung des ersten Antriebskranzes mit dem ersten Ritzel bereitstellt und ein zweiter Antriebsriemen die Wirkverbindung des zweiten Antriebskranzes mit dem zweiten Ritzel ermöglicht.

Als Antriebsriemen können Flach-, Rund-, Keil-, Keilrippen- oder Zahnriemen gewählt werden, wobei das Umfangsprofil der als Riemenscheiben ausgebildeten Antriebskränze und der Ritzel entsprechend ein Flach-, Rund-, Keil-, Keilrippen- oder Zahnprofil ist. Zahnriemen können bevorzugt sein, da durch den Formschluss zwischen Zahnriemen und Zahnprofil an den Riemenscheiben nicht nur hohe Kräfte bei geringerer Vorspannung übertragen werden können, sondern vor allem Schlupf verhindert wird und damit eine exakte Steuerung gewährleistet ist.

Bei einem zum Riemenantrieb alternativen Kettenantrieb sind die Antriebskränze der Doppelräder bzw. Hohl-Doppelräder und die Ritzel entsprechend als Kettenräder für eine Antriebskette ausgebildet, wobei eine erste Antriebskette die Wirkverbindung des ersten Antriebskranzes mit dem ersten Ritzel und eine zweite Antriebskette die Wirkverbindung des zweiten Antriebskranzes mit dem zweiten Ritzel bereitstellen. Ein Riemenantrieb kann gegenüber einem Kettenantrieb wegen geringerer Kosten und größerer Laufruhe bevorzugt sein.

Ferner kann für das Lenkgetriebe in einer weiteren erfindungsgemäßen Ausführungsform zur Ansteuerung der Taumelscheibe vorgesehen sein, dass die Taumelscheibe mit einem dritten Zahnrad gekoppelt ist, das mit den beiden Kegelradkränzen der beiden Doppelräder bzw. Hohl-Doppelräder in Eingriff steht und dessen Drehachse D in einem rechten Winkel zu der gemeinsamen Achse A der Doppelräder steht. Auf diese Weise können die Stellbewegungen der Antriebe über die (Hohl-) Doppelräder auf das dritte Zahnrad und von diesem auf die Taumelscheibe übertragen werden, die dadurch um die beiden Drehachsen A und D verkippt bzw. verschwenkt werden kann. Vorzugsweise kann die Taumelscheibe ferner mit einem vierten Zahnrad gekoppelt sein, das mit den beiden Kegelradkränzen der beiden Doppelräder bzw. Hohl-Doppelräder gekoppelt und auf der abgewandten Seite von dem dritten Zahnrad auf dessen Drehachse D angeordnet ist.

Die Erfindung bezieht sich ferner auf ein chirurgisches Instrument. Gemäß einer ersten Ausführungsform des chirurgischen Instruments weist es einen Schaft, eine am proximalen Ende des Schaftes angeordnete Betätigungseinheit und ein am distalen Ende des Schaftes angeordnetes Werkzeug auf. Das Werkzeug hat eine Werkzeugspitze, die mittels einer distalen Abwinkelungsmechanik abgewinkelt werden kann. Die Abwinkelungsmechanik kann durch eine mittels zweier Antriebe räumlich ausrichtbare Taumelscheibe gesteuert bzw. ausgerichtet werden, wozu das chirurgische Instrument ein erfindungsgemäßes Lenkgetriebe aufweist, wobei die beiden Antriebe Teil des erfindungsgemäßen Lenkgetriebes sind, das dazu ausgebildet ist, die Stellwinkel der zwei Antriebe auf die räumliche Ausrichtung der Taumelscheibe zu übertragen, um so die Abwinkelungsmechanik zu steuern.

Durch das erfindungsgemäße Lenkgetriebe kann das chirurgische Instrument konstruktiv einfach und platzsparend aufgebaut werden, so dass eine einfache Verbindung zu einem Roboter-Arm ermöglicht werden kann, bei der die Bewegung der Antriebe linear auf die Werkzeugspitze übertragen werden kann. Folge ist eine exakt steuerbare Verwendung des chirurgischen Instruments.

Um die räumlich verstellbare Taumelscheibe trotz der drehfesten Kopplung mit dem dritten Zahnrad, das mit den beiden Kegelradkränzen der beiden Doppelräder in Eingriff steht, dreidimensional verstellen zu können, d. h. die Kipp- bzw. Schwenkbewegungen mit einer Rotation der Taumelscheibe um die Längsachse B überlagern zu können, kann eine bevorzugte Ausführungsform des chirurgischen Instruments vorsehen, dass die Taumelscheibe um die Längsachse B des Schaftes über einen Lagerring rotierbar in einem Lenkring gelagert ist, der drehfest mit dem dritten Zahnrad gekoppelt ist. Zur rotativen Kopplung der Taumelscheibe mit einer koaxial zu einer Längsachse B des Schaftes verlaufenden Hauptwelle kann die Taumelscheibe kardanisch mit der Hauptwelle gekoppelt sein. Somit kann die Werkzeugspitze mittels der Taumelscheibe zusätzlich zum Verschwenken bzw. Verkippen durch die beiden Antriebe durch die Hauptwelle relativ zur Längsachse des Schaftes um die Längsachse des Schaftes rotiert werden.

Zur Ausbildung der kardanischen Lagerung der räumlich verstellbaren Taumelscheibe kann eine Ausführungsform des erfindungsgemäßen chirurgischen Instruments vorsehend, dass die Taumelscheibe über zwei um 180 ° versetzt zueinander angeordnete Lagerstifte verschwenkbar auf einer Kreuzgelenkscheibe gelagert ist, wobei die Kreuzgelenkscheibe über zwei um 180 ° versetzt zueinander angeordnete Lagerstifte verschwenkbar auf der Hauptwelle gelagert ist, und wobei die Lagerstifte der Taumelscheibe und der Kreuzgelenkscheibe um 90 ° versetzt zueinander angeordnet sind. Die kardanische Aufhängung ermöglicht eine Bewegungsführung in allen drei Raumachsen, wodurch die Werkzeugspitze gezielt gesteuert werden kann. Alternativ zu einer Kreuzgelenkscheibe mit zwei rechtwinklig gekreuzten Stift-Paaren zur kardanischen Lagerung der Taumelscheibe auf der Hauptwelle kann eine vorteilhafte Ausführungsform vorsehen, dass zur kardanischen Lagerung die Hauptwelle zwei in ihrer Außenfläche vorliegende Führungsnuten aufweist, die sich diametral und längs der Hauptwelle erstrecken, wobei die Taumelscheibe, die kreisringförmig mit einer Außenseite und einer Innenseite ausgebildet ist, zwei diametral und radial nach innen weisend an der Taumelscheibe angeordnete Stifte aufweist. Jeder der zwei fest an bzw. in der Taumelscheibe montierten Stifte greift in eine der beidseitig in die Hauptwelle eingebrachten Führungsnuten ein, sodass ein Drehwinkel der Welle auf die Taumelscheibe übertragbar ist. Vorteilhaft ergibt sich so eine drehsteife Verbindung zwischen Hauptwelle und Taumelscheibe, die auch bei einem großen Winkelversatz (± 40 ° und mehr) und Axialversatz eine Drehwinkelübertragung erlaubt, und dabei sehr kompakt aufgebaut, sowie einfach herzustellen und zu montieren ist. Grundsätzlich können allerdings auch eine Bogenzahnkupplung trotz eines relativ geringen Winkelversatzes, ein Gleichlaufgelenk trotz der aufwändigen Fertigung und komplexen Montage oder eine stoffschlüssige Kupplung, die häufig mit einer spielbehafteten Drehwinkelübertragung verbunden ist, zur kardanischen Lagerung einer Taumelscheibe auf einer Hauptwelle eingesetzt werden.

In einer weiteren Ausführungsform des erfindungsgemäßen chirurgischen Instruments verlaufen in Längsrichtung des Schaftes Lenkdrähte, die mit der Taumelscheibe des Lenkgetriebes verbunden sind. Vorzugsweise können die Lenkdrähte an der Taumelscheibe lösbar befestigt sein, beispielsweise mittels einer Klemmverbindung, damit in einem Fall der Beschädigung die Lenkdrähte einfach ausgetauscht werden können. Da die Taumelscheibe rotatorisch mit der Hauptwelle gekoppelt ist und durch den Lagerring drehbar in dem Lenkring gelagert ist, der drehfest mit dem dritten Zahnrad gekoppelt ist, wird bewirkt, dass weiter vorteilhaft beim Verschwenken der Werkzeugspitze relativ zur Längsachse und Rotieren um die Längsachse des Schaftes das Verdrillen der Lenkdrähte verhindert wird.

Ferner sieht eine noch weitere Ausführungsform des erfindungsgemäßen chirurgischen Instruments vor, dass das vierte Zahnrad mit der Taumelscheibe über einen Lagerring mit dem Lenkring gekoppelt ist, wobei das vierte Zahnrad gegenüber dem dritten Zahnrad frei drehbar ist. Dieses vierte Zahnrad schließt die umlaufende Verzahnungskette und sorgt so für eine gleichmäßig umlaufende und spielfreie Kraftverteilung.

In einer weiteren Ausführungsform des erfindungsgemäßen chirurgischen Instruments ist ein Betätigungselement axial verschiebbar in dem Schaft (und der Hauptwelle) gelagert und steht proximalseitig mit der Betätigungseinheit in Wirkverbindung. Die distale Abwinkelungsmechanik der abwinkelbaren Werkzeugspitze besteht aus an dem distalen Ende des Schaftes angeordneten Schwenkgliedern, die über die in Längsrichtung des Schaftes verlaufenden Lenkdrähte mit der Taumelscheibe verbunden sind.

In einer weiteren Ausführungsform des erfindungsgemäßen chirurgischen Instruments ist ein radialer Abstand der Lenkdrähte von der Längsachse des Schaftes an der Taumelscheibe größer als am proximalen Ende des Schaftes, aus dem die Lenkdrähte austreten. Dabei können die Lenkdrähte sich von dem proximalen Ende des Schaftes direkt zu der Taumelscheibe erstrecken, wobei die Lenkdrähte unter einem von 90° abweichenden Winkel zur Taumelscheibe verlaufen. Alternativ kann distalseitig vor der Taumelscheibe eine Fächerscheibe auf der Hauptwelle angeordnet sein, die den radialen Abstand der aus dem proximalen Schaftende austretenden Lenkdrähte von der Längsachse des Schaftes vergrößert, sodass die Lenkdrähte zwischen der Fächerscheibe und der Taumelscheibe annährend parallel zueinander verlaufen und in Bezug auf eine Scheibenfläche der Taumelscheibe einen Winkel von ca. 90° bilden. Aufgrund des geringeren Bauraumbedarfs kann die Variante ohne Fächerscheibe bevorzugt sein. Durch die Vergrößerung des radialen Abstandes der Lenkdrähte von der Längsachse des Schaftes, von beispielsweise einem Durchmesser von 4 mm auf einen Durchmesser von 18 mm, werden nicht nur die Montage und Fertigung des mit der räumlich verstellbaren Scheibe ausgestatten Antriebs der Lenkdrähte vereinfacht, sondern auch die Verstellwinkel der räumlich verstellbaren Scheibe bzw. infolge des vergrößerten Hebels die zur Abwinkelung benötigten Kräfte verringert, um einen dem Maß der Durchmesservergrößerung entsprechenden Verschwenkwinkel der Werkzeugspitze zu erzielen

Um beim Verschwenken des dritten und vierten Zahnrads relativ zur Längsachse des Schaftes eine Kollision der Zahnräder mit den Lenkdrähten und gegebenenfalls dem Betätigungselement zu vermeiden, können in den Zahnkränzen des dritten Zahnrads und des vierten Zahnrads Aussparungen für die Lenkdrähte und das Betätigungselement ausgebildet sein.

Das erfindungsgemäße chirurgische Instrument hat den Vorteil, dass viele dünne Lenkdrähte zur Ansteuerung der verschwenkbaren Werkzeugspitze verwendet werden können und diese Ansteuerung aufgrund des motorisierten Antriebs für die räumlich verstellbare Scheibe, an der die Lenkdrähte proximalseitig gelagert sind, feinfühlig, exakt und reproduzierbar erfolgt.

Weitere Ausführungsformen sowie einige der Vorteile, die mit diesen und weiteren Ausführungsformen verbunden sind, werden durch die nachfolgende ausführliche Beschreibung unter Bezug auf die begleitenden Figuren deutlich und besser verständlich. Gegenstände oder Teile derselben, die im Wesentlichen gleich oder ähnlich sind, können mit denselben Bezugszeichen versehen sein. Die Figuren sind lediglich eine schematische Darstellung einer Ausführungsform der Erfindung. In den Zeichnungen ist ein Ausführungsbeispiel der Erfindung dargestellt. Die Zeichnungen und die Beschreibung enthalten zahlreiche Merkmale in Kombination. Der Fachmann wird die Merkmale zweckmäßigerweise auch einzeln betrachten und zu sinnvollen weiteren Kombinationen unter Einhaltung des Geltungsbereichs der Ansprüche zusammenfassen.

Dabei zeigen:
- Fig. 1: eine schematische perspektivische Seitenansicht eines erfindungsgemäßen medizinischen Instruments,
- Fig. 2: eine perspektivische Ansicht des Antriebs gemäß Fig. 1 in einer ersten Ausführungsform,
- Fig. 3: eine Rückansicht auf den Antrieb gemäß Fig. 2,
- Fig. 4: eine Draufsicht auf den Antrieb gemäß Fig. 2,
- Fig. 5: eine Draufsicht auf eine Variante des Antriebs gemäß Fig. 2,
- Fig. 6: eine Draufsicht auf eine weitere Variante des Antriebs gemäß Fig. 2,
- Fig. 7: eine perspektivische Ansicht des Antriebs gemäß Fig. 1 in einer zweiten Ausführungsform,
- Fig. 8: eine Draufsicht auf den Antrieb gemäß Fig. 7,
- Fig. 9: eine perspektivische Ansicht des Antriebs gemäß Fig. 1 in einer dritten Ausführungsform,
- Fig. 10: eine perspektivische, teilweise geschnittene Detaildarstellung des Taumelscheibengetriebes.

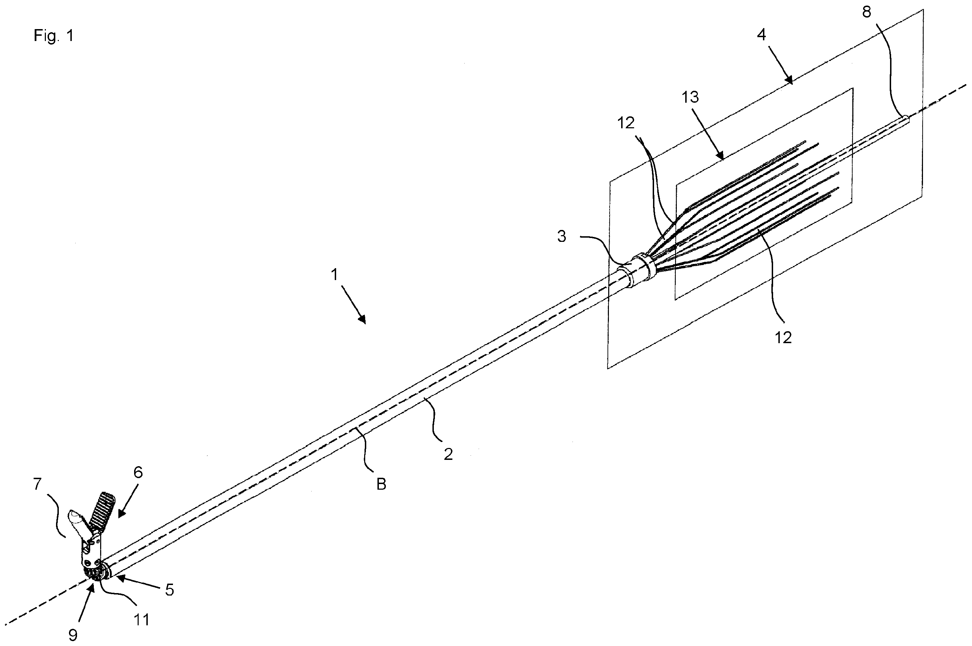

In Fig. 1 ist ein chirurgisches Instrument 1 mit einem hohlen Schaft 2 gezeigt, das eine am proximalen Ende 3 des Schaftes 2 angeordnete, nur schematisch dargestellte Betätigungseinheit 4 und eine am distalen Ende 5 des Schaftes 2 angeordnete Werkzeugspitze 6 aufweist. Die Werkzeugspitze 6 ist mit einem Instrument 7 verbunden, das über ein axial verschiebbar im Schaft 2 gelagertes Betätigungselement 8 betätigbar ist, das proximalseitig mit der Betätigungseinheit 4 in Wirkverbindung steht. Bei der Betätigungseinheit 4 kann es sich um eine manuell betätigbare Handhabe oder aber um eine für den robotischen Einsatz ausgelegte, also auch ohne manuelles Zutun betätigbare Baueinheit handeln.

Bei dem Instrument 7 der Werkzeugspitze 6 kann es sich beispielsweise um ein mit Maulteilen versehenes Werkzeug, wie in Fig. 1 dargestellt, oder aber um ein Endoskop, einen Applikator oder dergleichen handeln.

Die Werkzeugspitze 6 ist über einen Gelenkmechanismus 9 relativ zur Längsachse 10 des Schaftes 2 verschwenkbar, wobei der Gelenkmechanismus 9 aus am distalen Ende des Schaftes 5 angeordneten Schwenkgliedern 11 besteht, die über in Längsrichtung B des Schaftes 2 verlaufende Lenkdrähte 12 so mit einem am proximalen Ende 3 des Schaftes 2 angeordneten Antrieb bzw. Lenkgetriebe 13 verbunden sind, dass eine Bewegung des proximalseitigen Lenkgetriebes 13 eine entsprechende relative Bewegung der distalseitigen Schwenkglieder 11 und somit ein Verschwenken der Werkzeugspitze 6 verursacht.

Auch wenn voranstehend und nachfolgend nur der Begriff Lenkdrähte 12 verwendet wird, können funktional auch Lenkseile verwendet werden, weshalb der verwendete Begriff Lenkdrähte 12 synonym auch als Lenkseil zu lesen und zu verstehen ist.

Das axialverschiebbar im Schaft 2 gelagerte Betätigungselement 8 zum Betätigen des beispielsweise aus zwei Maulteilen bestehenden Instruments 7 ist bei den dargestellten Ausführungsformen als Zug-/Schubstange ausgebildet.

Das Lenkgetriebe 13 für die Lenkdrähte 12 ist bei dem in den Abbildungen dargestellten und nachfolgend beschriebenen medizinischen Instrument 1 als motorisierter Antrieb 13 ausgebildet.

In noch einer weiteren Ausführungsform des erfindungsgemäßen chirurgischen Instruments ist die Taumelscheibe um die Längsachse B des Schaftes rotierbar in einem Lenkring gelagert. Dazu ist ein Lagerring in dem Lenkring vorgesehen, der drehfest mit dem dritten Zahnrad gekoppelt ist. Dies dient dazu, dass die Werkzeugspitze zusätzlich zum Verschwenken relativ zur Längsachse des Schaftes auch um die Längsachse des Schaftes rotiert werden kann, ohne dass die Lenkdrähte verdrillt werden.

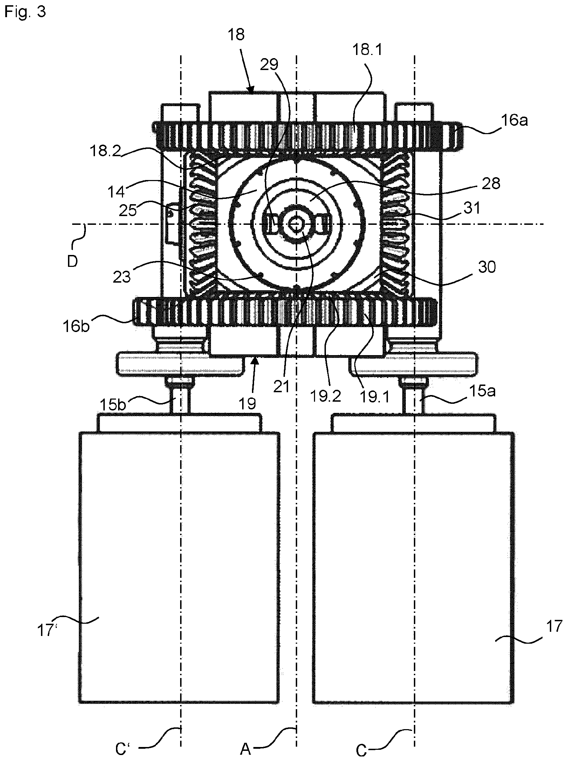

Kernstück des Antriebs 13 ist eine räumlich verstellbare Scheibe bzw. Taumelscheibe 14 (Fig. 3, 9, 10), an der die Lenkdrähte 12 so gelagert sind, dass eine Verlagerung der Taumelscheibe 14 über die an der Taumelscheibe 14 gelagerten Lenkdrähte 12 ein Verschwenken der Werkzeugspitze 6 bewirkt. Mittels des motorisierten Antriebs bzw. Lenkgetriebes 13 kann die Taumelscheibe 14 verlagert werden.

Durch die Verwendung eines motorisierten Antriebs 13 für die Taumelscheibe 14 ist es möglich, die Lenkdrähte 12 zum Verschwenken der distalseitigen Schwenkglieder 11 bzw. der Werkzeugspitze 6 exakt, feinfühlig in kleinsten Schritten und auch reproduzierbar anzusteuern. Darüber hinaus ist die Anzahl der zu verwendenden Lenkdrähte 12 für einen motorisierten Antrieb 13 recht frei wählbar.

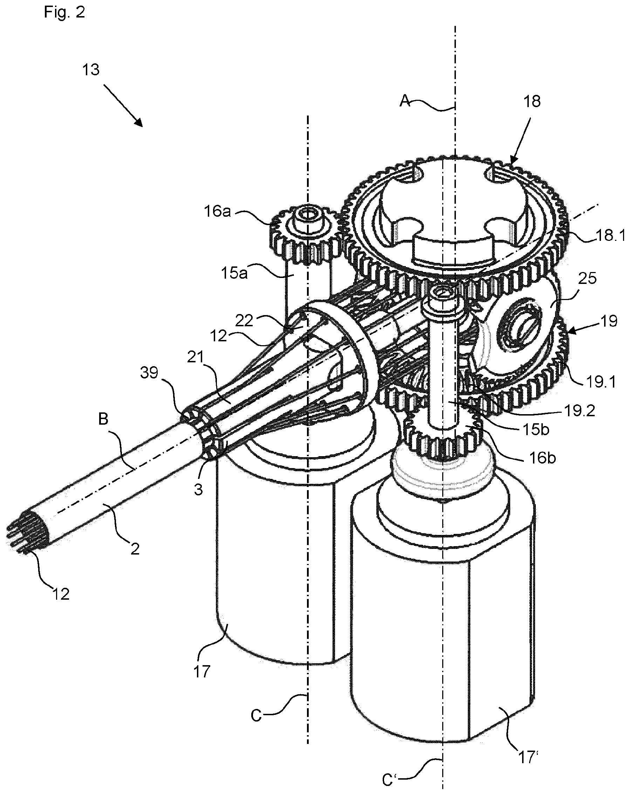

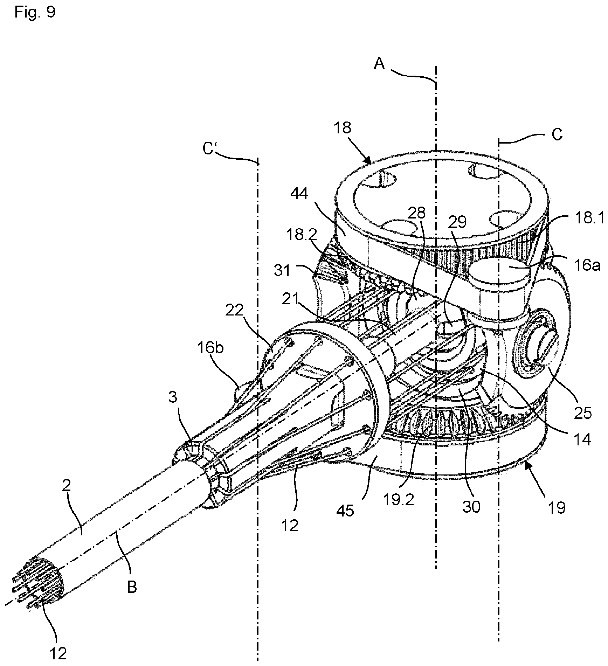

Das Lenkgetriebe 13 weist, wie in Fig. 2 bis 9 zu sehen ist, zwei Antriebe mit Motoren 17, 17' (in Fig. 9 nicht dargestellt) mit parallelen Antriebsachsen C, C' auf, die ferner parallel zu einer gemeinsamen Drehachse A der zwei Antriebsräder 18, 19 verlaufen, zwischen denen die räumlich verstellbare Taumelscheibe 14 angeordnet ist.

Fig. 2 und 3 zeigen ein Beispiel für eine erste Ausführungsform des erfindungsgemäßen Lenkgetriebes 13 für ein chirurgisches Instrument 1, das wie in Fig. 1 dargestellt, am proximalen Ende 3 des Schafts 2 angeordnet ist, der die Längs- bzw. Hauptachse B definiert und am distalen Ende 5 die Abwinkelungsmechanik 9 aufweist. Die Antriebe des Lenkgetriebes 13 weisen ein von dem ersten Motor 17 angetriebenes erstes Antriebsrad 18 und ein von dem zweiten Motor 17' angetriebenes zweites Antriebsrad 19 auf, zwischen denen die Taumelscheibe 14 angeordnet ist. Die beiden Antriebsräder 18, 19 liegen auf einer gemeinsame Drehachse A und sind beide als Doppelräder 18, 19 ausgebildet, sodass sie jeweils einen Kegelradkranz 18.2, 19.2 und einen Antriebskranz 18.1, 19.1 aufweisen. Vorzugsweise können die Doppelräder 18, 19 einstückig gefertigt sein. Alternativ ist aber ebenso möglich, dass jedes Doppelrad aus jeweils einem Kegelrad und einem damit verbundenen Rad mit Antriebskranz besteht.

Die Kegelradkränze 18.2, 19.2 der Doppelräder 18, 19 sind einander zugewandt und kämmen mit einem dritten Zahnrad 25 und einem diesem gegenüber liegend angeordneten vierten Zahnrad 31, die mit der Taumelscheibe 14 gekoppelt sind und auf einer Drehachse D liegen, die in einem rechten Winkel zu der gemeinsamen Achse A der Doppelräder 18, 19 steht.

Um die Stellbewegungen der Motoren 17, 17' auf die Antriebsräder 18, 19 zu übertragen, weist das Lenkgetriebe 13 ein erstes Ritzel 16a auf, das über eine erste Antriebswelle 15a von dem ersten Motor 17 angetrieben wird, und mit dem Antriebskranz 18.1 des ersten Doppelrads 18 kämmt. Entsprechend wird das zweite Ritzel 16b, das mit dem Antriebskranz 19.1 des zweiten Doppelrads 19 kämmt, über eine zweite Antriebswelle 15b von dem zweiten Motor 17' angetrieben. Die Antriebswellen 15a und 15b definieren die erste und zweite Antriebsachse C und C', die nicht nur parallel zueinander, sondern auch parallel zu der gemeinsamen Drehachse A der zwei Antriebsräder 18, 19 verlaufen.

Mit dieser Anordnung können die zwei angetriebenen Doppelräder 18, 19 des Lenkgetriebes 13 derart in Bezug auf die Taumelscheibe 14 angeordnet werden, dass die zwei Antriebsachsen C, C' und die gemeinsame Drehachse A der zwei Doppelräder 18, 19 senkrecht, d. h. im rechten Winkel zu der Längsachse B des chirurgischen Instruments 1 verlaufen. Auf diese Weise können die Antriebsachsen C, C' relativ nahe an Längsachse B positioniert werden. Da durch das Lenkgetriebe 13 die Motoren 17, 17' nicht gegenüber liegend verbaut werden müssen, um die beiden Antriebsräder 18, 19 zu bewegen, kann die Bauhöhe des Instruments durch die parallele Anordnung beider Motoren 17, 17' nebeneinander auf einer Seite in Bezug zu der Längsachse B reduziert werden.

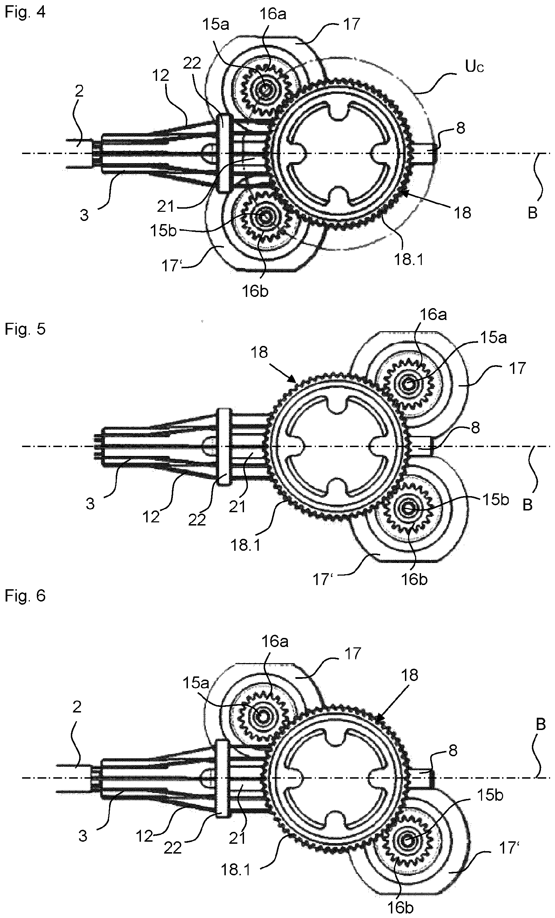

Die Positionierung der Antriebsachsen C, C' auf einer Kreisbahn U_{C} um die Antriebsräder 18, 19 ist dabei nahezu beliebig wählbar, wie in Fig. 4-6 angedeutet ist, sofern der Bauraum dort nicht durch andere Instrumentenkomponenten wie z. B. Hauptwelle 21, Lenkdrähte 12, Fächerscheibe 22 oder Betätigungselement 8 belegt ist. Die in den Fig. 4-6 gezeigten Varianten zeigen Anordnungen der Antriebseinheiten aus Ritzel 16a, 16b, Antriebswellen 15a, 15b und Motoren 17, 17' möglichst nahe an der Längsachse B, die aus Gründen der Bauraumersparnis bevorzugt sind. Denkbar ist jedoch auch eine Anordnung der Antriebseinheiten an jeder beliebigen Stelle der Kreisbahn U_{C}, solange die Ritzel 16a, 16b mit den Antriebskränzen 18.1, 19.1 kämmen. Der Durchmesser der Kreisbahn U_{C}, hängt von dem Durchmesser der Antriebsräder 18, 19 und dem Durchmesser der Ritzel 16a, 16b bzw. der Verzahnung ab. Durch Änderung des Übersetzungsverhältnisses kann sich daher ein geänderter Kreisbahndurchmesser ergeben, der die mögliche Positionierung der Antriebsachsen C, C' vorgibt.

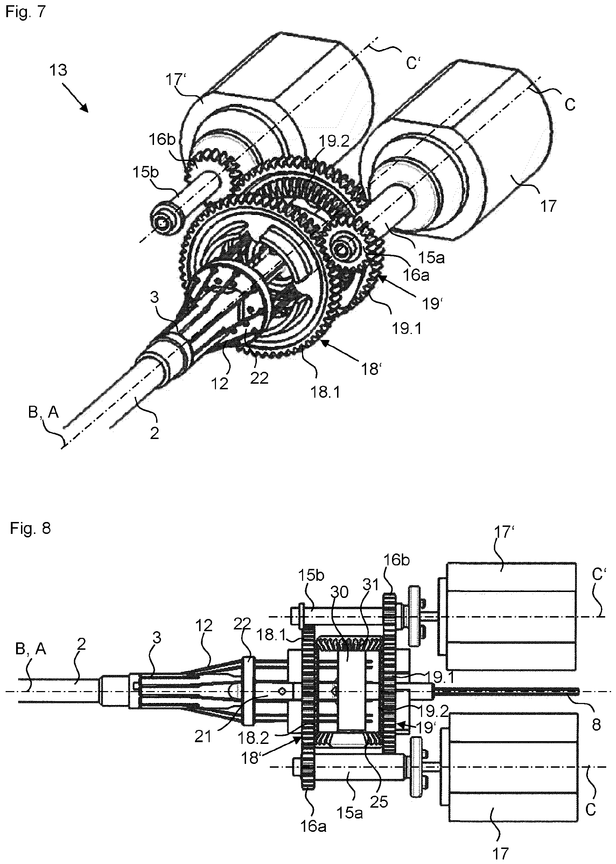

In Fig. 7 und 8 ist ein Beispiel für eine alternative Ausführungsform des Lenkgetriebes 13 dargestellt, bei dem die zwei Antriebsachsen C, C', die durch die Antriebseinheiten aus Motor 17, 17', Antriebswelle 15a, 15b, und Ritzel 16a, 16b definiert werden, parallel zu der Längsachse B des chirurgischen Instruments 1 verlaufen. Dies wird ermöglicht, indem die durch die Antriebseinheiten angetriebenen Doppelräder als Hohl-Doppelräder 18', 19' ausgeführt sind, und damit im Lenkgetriebe 13 in einer Weise in Bezug auf die Taumelscheibe 14 angeordnet werden können, dass die gemeinsame Drehachse A der zwei Hohl-Doppelräder 18', 19' mit der Längsachse B des chirurgischen Instruments 1 fluchtet. Durch die Aushöhlung der Doppelräder 18', 19' können die zur Taumelscheibe 14 führenden Instrumentenkomponenten wie z. B. Hauptwelle 21 und Lenkdrähte 12 durch die Doppelräder 18', 19' hindurchgeführt werden. Auch hierdurch wird eine bauraumsparende, längsachsnahe Positionierung der Antriebseinheiten ermöglicht, wobei auch hier eine Positionierung der Antriebsachsen C, C' auf einer Kreisbahn um die Doppelräder 18', 19' (entsprechend U_{C} in Fig. 4) grundsätzlich beliebig wählbar ist.

In den mit Fig. 2 bis 8 gezeigten Beispielen sind die Antriebskränze 18.1, 19.1 der Doppelräder 18, 19 bzw. Hohl-Doppelräder 18', 19' als Zahnkränze und die Ritzel 16a, 16b entsprechend als Zahnräder ausgeführt, die mit den als Zahnkränzen ausgebildeten Antriebskränzen 18.1, 19.1 in Eingriff stehen bzw. kämmen. Fig. 9 hingegen zeigt ein Beispiel für eine weitere alternative Ausführungsform des Lenkgetriebes 13 mit einem Riemengetriebe zur Übertragung der Stellbewegung des Motors 17, 17' auf das jeweilige Antriebsrad 18, 19.

In Fig. 9 sind die Motoren aus Gründen der besseren Übersichtlichkeit nicht dargestellt, es versteht sich aber von selbst, dass die dargestellten, als Riemenscheiben ausgeführten Ritzel 16a, 16b von einem jeweiligen Motor mit einer jeweiligen Antriebswelle, die auf den Antriebsachsen C, C' liegen angetrieben werden. Auch die Antriebskränze 18.1, 19.1 der Doppelräder 18, 19 sind als Riemenscheiben ausgeführt, sodass eine Stellbewegung vom ersten Ritzel 16a über einen ersten Antriebsriemen 44 auf das erste Antriebsrad 18 übertragen werden kann, und entsprechend eine Stellbewegung vom zweiten Ritzel 16b über einen zweiten Antriebsriemen 45 auf das zweite Antriebsrad 19.

Wie in Fig. 9 am Antriebskranz 18.1 erkennbar, der eine Zahnung aufweist, ist in diesem Beispiel der Riemen 44 als Zahnriemen ausgeführt. Folglich weist auch das als Riemenscheibe ausgebildete Ritzel 16a eine Zahnung auf. Entsprechendes gilt für die zweite Antriebseinheit, d. h., Ritzel 16b, Riemen 45 und Antriebskranz 19.1, auch wenn dies in der Figur nicht ersichtlich ist.

Selbstverständlich sind auch von einem Zahnriemengetriebe abweichende Riemengetriebe, z. B. Flachriemen, Rundriemen, Keilriemen oder Keilrippenriemen, einsetzbar, wobei die Riemenscheiben mit einem dem Antriebsriemen entsprechenden Umfangsprofil ausgebildet sind. Analog zu dem in Fig. 9 gezeigten Beispiel mit Riemengetriebe ist auch eine Ausführungsform mit einem Kettengetriebe denkbar (nicht figurativ dargestellt), bei dem anstelle der in Fig. 9 gezeigten Riemen 44, 45 Ketten eingesetzt werden und entsprechend die Ritzel 16a, 16b sowie die Antriebskränze 18.1, 19.1 als Kettenräder ausgeführt sind
Bei den Ausführungen mit Antriebsriemen oder entsprechend Antriebskette ergibt sich, dass die Positionierung der Ritzel 16a, 16b bzw. der Antriebsachsen C, C' nicht nur unabhängig voneinander und beliebig auf einer Kreisbahn um die Antriebsräder 18, 19, sondern durch Variation der Länge des Antriebsriemens bzw. der Kette auch in beliebigem Abstand von den Antriebsrädern 18, 19 gewählt werden kann. Da jedoch eine Bauraumreduktion erstrebenswert ist, ist eine Positionierung der Antriebsachsen C, C' nahe der Antriebsräder 18, 19, und vorzugsweise auch nahe der Längsachse B bevorzugt.

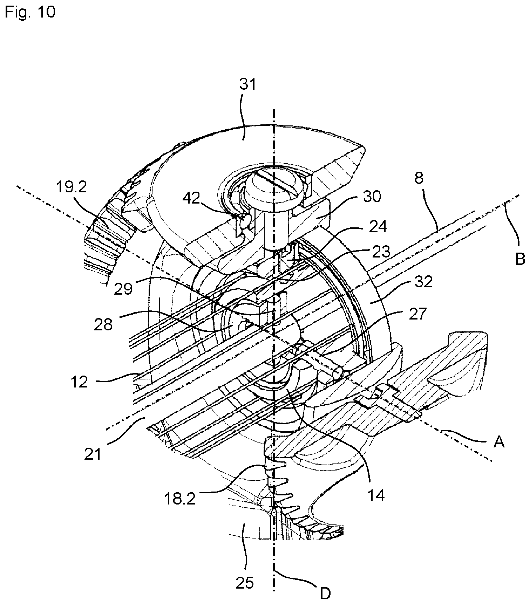

Der nachfolgend beschriebene Aufbau der räumlich verstellbaren Scheibe 14 und deren Lagerung sind bei allen Ausführungsformen des motorisierten Antriebs bzw. Lenkgetriebes 13 identisch. Der Aufbau und der Betrieb des Lenkgetriebes 13 sowie insbesondere der über die Antriebseinheiten betätigbaren Taumelscheibe 14 werden nachfolgend anhand Fig. 3, 8, 9 und insbesondere Fig. 10 beschrieben, in der aus Gründen der besseren Übersichtlichkeit nur die Kegelräder mit den Kegelradkränzen 18.2, 19.2 der Antriebsräder 18, 19 dargestellt sind.

Wie aus den Abbildungen ersichtlich, ist im Schaft 2 eine sich koaxial zur Längsachse B des Schaftes 2 erstreckende hohle Hauptwelle 21 angeordnet, die um die Längsachse B des Schaftes 2 rotierbar ist und sich über das proximale Ende 3 des Schaftes 2 hinaus bis in den Bereich des Lenkgetriebes 13 erstreckt. Innerhalb dieser hohlen Hauptwelle 21 ist axialverschiebbar das Betätigungselement 8 zur Betätigung des Instruments 7 gelagert.

Die am proximalen Ende 3 des Schaftes 2 aus dem Schaft 2 austretenden Lenkdrähte 12, wofür am proximalen Schaftende ein Schaftendstück 3 vorgesehen sein kann, in dem Durchtrittschlitze für die Lenkdrähte 12 vorgesehen sind, werden im dargestellten Beispiel über eine drehfest in Bezug auf der Hauptwelle 21 an dem Schaftendstück 3 angeordnete Fächerscheibe 22 aufgefächert, wodurch der radiale Abstand der Lenkdrähte 12 von der Längsachse B des Schaftes 2 vergrößert wird. Während der Durchmesser des die Längsachse B des Schaftes 2 koaxial umgebenden Bündels der Lenkdrähte 12 innerhalb des Schaftes 2 bzw. an dem distalen Ende 5 im Bereich der Abwinkelungsmechanik 9 beispielsweise 4 mm beträgt, beträgt der Durchmesser des von den Lenkdrähten 12 gebildeten Bündels hinter der Fächerscheibe 22 beispielsweise 18 mm. Durch die mit Hilfe der Fächerscheibe 22 erzielte Vergrößerung des radialen Abstandes der Lenkdrähte 12 von der Längsachse B des Schaftes 2 werden nicht nur die Montage und Fertigung des mit der Taumelscheibe 14 ausgestatten Getriebes 13 vereinfacht, sondern wird auch der notwendige Verstellwinkel der Taumelscheibe 14 proportional verringert, um einen erwünscht hohen Verschwenkwinkel der Werkzeugspitze 6 zu erzielen. Mit dieser beispielhaften Vergrößerung des Durchmessers des Lenkdrahtbündels von 4 mm innerhalb des Schaftes 2 auf 18 mm hinter der Fächerscheibe 22 verringert sich ein Verstellwinkel der Taumelscheibe 14 entsprechend um das 4,5-fache gegenüber dem am distalen Ende erzielbaren Verschwenkwinkel der Werkzeugspitze 6. Um diese um 90° abzuwinkeln, bedarf es somit nur einer Verschwenkung der Taumelscheibe 14 um 20°.

Proximalseitig hinter der Fächerscheibe 22 werden die parallel zur Längsachse B des Schaftes 2 verlaufenden Lenkdrähte 12 der Taumelscheibe 14 zugeführt. Zum Festlegen der Lenkdrähte 12 an der Taumelscheibe 14 sind in der Taumelscheibe 14 Durchgangsbohrungen 23 für jeden Lenkdraht 12 ausgebildet, wobei im dargestellten Beispiel die Lenkdrähte 12 innerhalb der Durchgangsbohrungen 23 über Madenschrauben 24 kraftschlüssig mit der Taumelscheibe 14 verbunden und fixiert sind. Dazu alternative Befestigungsformen der Lenkdrähte an der Taumelscheibe umfassen beispielsweise auch Schweißen oder Crimpen oder andere Klemmvorrichtungen.

Die Antriebsräder 18 und 19 sind mit einem dritten Zahnrad 25 gekoppelt, das vorzugsweise als Kegelrad ausgebildet ist und mit den beiden Kegelradkränzen 18.2, 19.2 der Doppelräder 18 und 19 in Eingriff steht, sodass die Drehachse D des dritten Zahnrads 25 die gemeinsame Drehachse A der Antriebsräder 18 und 19 sowie die Längsachse B des Schaftes 2 schneidet. Durch die drei miteinander kämmenden Zahnräder 18, 19 und 25 wird jede Bewegung der beiden Antriebsräder 18 und 19 direkt auf die mit dem dritten Zahnrad 25 gekoppelte Taumelscheibe 14 übertragen, was eine direkte Betätigung der Lenkdrähte 12 bewirkt.

Zur Ausbildung einer kardanischen Lagerung der Taumelscheibe 14 auf der Hauptwelle 21 ist die Taumelscheibe 14 über zwei um 180° versetzt zueinander angeordnete Lagerstifte 27 verschwenkbar auf einer Kreuzgelenkscheibe 28 gelagert, die wiederrum über zwei um 180° versetzt zueinander angeordnete Lagerstifte 29 verschwenkbar auf der Hauptwelle 21 gelagert ist. In Fig. 10 ist aufgrund der Teilschnittansicht jeweils nur ein Lagerstift 27 und ein Lagerstift 29 zu sehen.

Die Lagerstifte 27 der Taumelscheibe 14 und die Lagerstifte 29 der Kreuzgelenkscheibe 28 sind dabei um 90° versetzt zueinander angeordnet. Diese Lagerung ermöglicht es, die Taumelscheibe 14 um zwei rechtwinklig zueinander stehende Achsen relativ zur Längsachse B des Schaftes 2 zu verschwenken und eine Rotation der Hauptwelle 21 um die Längsachse B auf die Taumelscheibe 14 zu übertragen, wodurch über die Lenkdrähte 12 distalseitig die Werkzeugspitze 6 in alle Raumrichtungen relativ zur Längsachse B des Schaftes 2 verschwenkbar ist.

Eine nicht dargestellte, alternative kardanische Lagerung der Taumelscheibe 14 auf der Hauptwelle 21 sieht vor, dass die Hauptwelle 21 in dem zur Lagerung der Taumelscheibe 14 vorgesehenen Bereich zwei sich längs der Hauptwelle 21 erstreckende und beidseitig bzw. diametral in der Hauptwelle 21 eingebrachte Führungsnuten aufweist, in die zwei diametral und radial nach innen weisend an der Taumelscheibe 14 angeordnete Stifte eingreifen. Durch diesen Eingriff kann die Taumelscheibe 14 aus einer neutralen Position, in der die Taumelscheibe 14 in einer durch die Drehachse A definierten Ebene senkrecht, d. h. rechtwinklig, zur Längsachse B liegt, sowohl um Drehachse D als auch um Drehachse A verschwenkt werden. Überlagerte Bewegungen durch Verschwenken um beide Drehachsen A, D, sind ebenfalls möglich. Ferner gestattet der Eingriff der Stifte 29 in die Führungsnuten 20a die Übertragung eines Drehwinkels der Hauptwelle 21 auf die Taumelscheibe 14, sodass die Taumelscheibe 14 dreidimensional relativ zur Längsachse B des Schaftes 2 verlagert werden kann. Die maximale Verkippung bzw. Verdrehung bzw. die maximalen Kipp- und Drehwinkel um Drehachse A und D sind durch die Länge und Tiefe der Führungsnuten 20a im Zusammenwirken mit Innendurchmesser und Stärke der Taumelscheibe 14 und Länge der Stifte 29 bestimmt. Im dargestellten Beispiel weist die Hauptwelle 21 in dem zur Lagerung der Taumelscheibe 14 vorgesehenen Bereich einen Kugelabschnitt 20b auf, an dem die Führungsnuten 20a vorliegen und der die Taumelscheibe 14 in axialer Richtung festlegt. Die Taumelscheibe 14 weist hierbei eine an den Kugelabschnitt 20b angepasst konturierte Aufnahmeausnehmung auf.

Wie weiterhin aus Fig. 3, 8, 9 und 10 ersichtlich, ist die räumlich verstellbare Scheibe 14 in einem drehfest mit dem dritten Zahnrad 25 gekoppelten Lenkring 30 gelagert.

Um die durch die Antriebsräder 18, 19 und Zahnrad 25 gebildete Verzahnungskette zu einem geschlossenen Verzahnungsring zu schließen, der eine gleichmäßig umlaufende Kraftverteilung gewährleistet, ist auf der Drehachse D des dritten Zahnrads 25 gegenüber liegend zum dritten Zahnrad 25 ein viertes Zahnrad 31 angeordnet, das ebenfalls vorzugsweise als Kegelrad ausgebildet ist mit den Kegelradkränzen 18.2, 19.2 der beiden Doppelräder 18 und 19 in Eingriff steht.

Die Taumelscheibe 14 ist über einen Lagerring 32 in dem drehfest mit dem dritten Zahnrad 25 gekoppelten Lenkring 30 gelagert, um eine Rotation der Taumelscheibe 14 um die Längsachse B des Schaftes 2 zu ermöglichen. Der drehfest mit dem dritten Zahnrad 25 gekoppelte Lenkring 30 ist durch eine Lagerung mittels Lagerring 42 frei in Bezug auf das vierte Zahnrad 31 drehbar, so dass eine Rotation des vierten Zahnrads 31 um seine Drehachse D keine Verdrehung des Lenkrings 30 und der Taumelscheibe 14 bewirkt.

Die beschriebene kardanische Lagerung der Taumelscheibe 14 auf der Hauptwelle 21 ermöglicht es, die Taumelscheibe 14 dreidimensional relativ zur Längsachse B des Schaftes 2 zu verlagern. Wenn ausgehend von der in Fig. 10 dargestellten neutralen Ausgangsstellung, in der die Taumelscheibe 14 senkrecht zur Längsachse B des Schaftes 2 ausgerichtet ist, die Antriebsräder 18 und 19 über die Motoren 17, 17' (vgl. Fig. 2-8) so angetrieben werden, dass sich die Antriebsräder 18 und 19 in dieselbe Richtung drehen, bewirkt diese Verdrehung der Antriebsräder 18 und 19 aufgrund des kämmenden Eingriffs mit dem dritten Zahnrad 25 und dem vierten Zahnrad 31 ein Verkippen der Baueinheit, die aus dem dritten Zahnrad 25, der mit dem dritten Zahnrad 25 gekoppelten Taumelscheibe 14 und dem vierten Zahnrad 31 gebildet wird, um die gemeinsame Drehachse A der Antriebsräder 18 und 19. Zur Vereinfachung der Funktionsbeschreibung wird nachfolgend auf die Ausrichtung der Lagerstifte 27, 29 der kardanischen Lagerung in Bezug auf die Drehachsen A und D genommen. Tatsächlich werden bei Rotation der Hauptwelle 21 und damit der Taumelscheibe 14 die Lagerstifte 27, 29 nicht mehr wie dargestellt mit den Achsen A und D fluchten, sodass die durch die Lagerstifte 27, 29 bereitgestellten Schwenkachsen der Taumelscheibe 14 von den Drehachsen A, D des Lenkgetriebes 13 abweichen können.

Die mit der Drehachse A der Antriebsräder 18 und 19 fluchtenden Lagerstifte 27, über die die Taumelscheibe 14 verschwenkbar an der Kreuzgelenkscheibe 28 gelagert ist, ermöglichen dieses Verkippen der Taumelscheibe 14 relativ zur Hauptwelle 21. Dieses Verkippen der Taumelscheibe 14 um die Drehachse A relativ zur Längsachse B des Schaftes 2 bewirkt über die Lenkdrähte 12, dass distalseitig die Werkzeugspitze 6 in entsprechender Weise relativ zur Längsachse B des Schaftes 2 verschwenkt wird.

Wenn ausgehend von der in Fig. 10 dargestellten neutralen Ausgangsstellung, in der die Taumelscheibe 14 senkrecht zur Längsachse B des Schaftes 2 ausgerichtet ist, die Antriebsräder 18 und 19 über die Motoren 17, 17' so angetrieben werden, dass sich die Antriebsräder 18 und 19 in entgegengesetzte Richtungen drehen, bewirkt diese Verdrehung der Antriebsräder 18 und 19 aufgrund des kämmenden Eingriffs mit dem dritten Zahnrad 25 ein Verdrehen der Baueinheit, die aus dem dritten Zahnrad 25, und der mit dem dritten Zahnrad 25 gekoppelten Taumelscheibe 14 gebildet wird, um die Drehachse D des dritten Zahnrads 25.

Die mit der Drehachse D des dritten Zahnrads 25 fluchtenden Lagerstifte 29, über die die Kreuzgelenkscheibe 28 verschwenkbar an der Hauptwelle 21 gelagert ist, ermöglichen zusammen mit der freien Drehbarkeit der Taumelscheibe 14 relativ zum vierten Zahnrad 31 aufgrund des Lagerrings 32 dieses Verdrehen der Kreuzgelenkscheibe 28 relativ zur Hauptwelle 21. Dieses Verdrehen der Taumelscheibe 14 um die Drehachse D relativ zur Längsachse B des Schaftes 2 bewirkt über die Lenkdrähte 12 das distalseitig dieWerkzeugspitze 6 in entsprechender Weise relativ zur Längsachse B des Schaftes 2 verschwenkt wird.

Ferner ist es selbstverständlich möglich, die beschriebenen Bewegungen zu überlagern, sodass beispielsweise die Taumelscheibe 14 um die gemeinsame Drehachse A der Antriebsräder 18, 19 verkippt und gleichzeitig auch noch um die Drehachse D des dritten Zahnrads 25 verdreht wird. Durch die Kombination der beiden Bewegungsabläufe aufgrund der einzeln ansteuerbaren Motoren 17, 17' des Getriebes 13 und die Kopplung mit der Hauptwelle 21 lässt sich die Taumelscheibe 14 dreidimensional relativ zur Längsachse B des Schaftes 2 verstellen, woraus aufgrund der Kopplung über die Lenkdrähte 12 eine entsprechende räumliche Verlagerung der Werkzeugspitze 6 resultiert.

Ein wie zuvor beschrieben ausgebildetes chirurgisches Instrument 1 zeichnet sich dadurch aus, dass viele dünne Lenkdrähte 12 zur Ansteuerung der verschwenkbaren Werkzeugspitze 6 verwendet werden können, und diese Ansteuerung aufgrund des motorisierten Antriebs 13 für die Taumelscheibe 14, an der die Lenkdrähte 12 gelagert sind, feinfühlig, exakt und reproduzierbar erfolgt.

In den Zeichnungen ist ein Ausführungsbeispiel der Erfindung dargestellt. Die Zeichnungen, die Beschreibung und die Ansprüche enthalten zahlreiche Merkmale in Kombination. Der Fachmann wird die Merkmale zweckmäßigerweise auch einzeln betrachten und zu sinnvollen weiteren Kombinationen zusammenfassen. Die vorliegende Erfindung stellt ein Lenkgetriebe 13 für ein chirurgisches Instrument 1 bereit, das am proximalen Ende 3 eines Schafts 2 anordenbar ist, der eine Längsachse B definiert und am distalen Ende 5 eine Abwinkelungsmechanik 9 aufweist, wobei das Lenkgetriebe 13 zwei motorisierte Antriebe aufweist und dazu ausgebildet ist, über die Stellwinkel der zwei Antriebe eine Taumelscheibe 14 räumlich auszurichten. Die beiden Antriebe weisen jeweils ein von Motoren 17, 17' angetriebenes Antriebsrad 18, 18', 19, 19' auf, das als Doppelrad 18, 18', 19, 19' mit einem Kegelradkranz 18.2, 19.2 und einem Antriebskranz 18.1, 19.1 ausgebildet ist. Dabei ist die Taumelscheibe 14 zwischen den zwei Antriebsrädern 18, 19; 18', 19', die eine gemeinsame Drehachse A aufweisen, angeordnet, und die Kegelradkränze 18.2, 19.2 sind einander zugewandt, wobei die Antriebskränze 18.1, 19.1 beider Doppelräder 18, 19; 18', 19' mit jeweils einem Ritzel 16a, 16b in Wirkverbindung stehen. Dabei sind die Ritzel 16a, 16b, die jeweils mit einem der Antriebskränze 18.1, 19.1 in Wirkverbindung stehen, über jeweils eine Antriebswelle 15a, 15b, die eine Antriebsachse C, C' definiert, durch den jeweiligen Motor 17, 17' antreibbar, wobei die zwei Antriebsachsen C, C' parallel zueinander und parallel zu der gemeinsamen Drehachse A der zwei Antriebsräder 18, 19; 18', 19' verlaufen. Ferner wird ein chirurgisches Instrument 1 offenbart, das ein solches Lenkgetriebe 13 aufweist.

### Bezugszeichenliste

- 1: Medizinisches Instrument
- 2: Schaft
- 3: Proximales Ende (Schaft)/ Endstück
- 4: Betätigungseinheit
- 5: Distales Ende (Schaft)
- 6: Instrumentenspitze
- 7: Instrument
- 8: Betätigungselement
- 9: Gelenkmechanismus
- 11: Schwenkglied
- 12: Lenkdraht
- 13: Lenkgetriebe
- 14: Scheibe (räumlich verstellbar)
- 15a,b: Antriebswelle
- 16a,b: Ritzel
- 17, 17': Motor
- 18, 18': Antriebsrad, hohles Antriebsrad
- 18.1, 18.2: Antriebskranz, Kegelradkranz
- 19, 19': Antriebsrad, hohles Antriebsrad
- 19.1, 19.2: Antriebskranz, Kegelradkranz
- 21: Hauptwelle
- 22: Fächerscheibe
- 23: Durchgangsbohrung
- 24: Madenschraube
- 25: Drittes Zahnrad
- 27: Lagerstift
- 28: Kreuzgelenkscheibe
- 29: Lagerstift
- 30: Lenkring
- 31: Viertes Zahnrad
- 32: Lagerring
- 42: Lagerring
- 44, 45: Antriebsriemen
- A: Gemeinsame Drehachse Antriebsräder
- B: Längsachse
- C, C': Antriebsachse
- D: Drehachse drittes Zahnrad
- U_{C}: Kreisbahn

## Patentansprüche

1. Lenkgetriebe (13) für ein chirurgisches Instrument (1), das am proximalen Ende (3) eines Schafts (2) anordenbar ist, der eine Längsachse (B) definiert und am distalen Ende (5) eine Abwinkelungsmechanik (9) aufweist,
wobei das Lenkgetriebe (13) zwei motorisierte Antriebe und eine Taumelscheibe (14) aufweist und dazu ausgebildet ist, über die Stellwinkel der zwei Antriebe die Taumelscheibe (14) räumlich auszurichten, die dazu ausgebildet ist, die distale Abwinkelungsmechanik (9) des chirurgischen Instruments (1) zu steuern,
wobei
der erste Antrieb ein von einem ersten Motor (17) angetriebenes erstes Antriebsrad (18, 18') aufweist, das als Doppelrad (18, 18') mit einem ersten Kegelradkranz (18.2) und einem ersten Antriebskranz (18.1) ausgebildet ist,
und der zweite Antrieb ein von einem zweiten Motor (17') angetriebenes zweites Antriebsrad (19, 19') aufweist, das als Doppelrad (19, 19') mit einem zweiten Kegelradkranz (19.2) und einem zweiten Antriebskranz (19.1) ausgebildet ist, wobei
die Taumelscheibe (14) zwischen den zwei Antriebsrädern (18, 19; 18', 19'), die eine gemeinsame Drehachse (A) aufweisen, angeordnet ist, und die Kegelradkränze (18.2, 19.2) einander zugewandt sind, und
wobei
die Antriebskränze (18.1, 19.1) beider Doppelräder (18, 19; 18', 19') mit jeweils einem Ritzel (16a, 16b) in Wirkverbindung stehen,
wobei ein erstes Ritzel (16a), das mit dem ersten Antriebskranz (18.1) in Wirkverbindung steht, über eine erste Antriebswelle (15a), die eine erste Antriebsachse (C) definiert, durch den ersten Motor (17) antreibbar ist, und
ein zweites Ritzel (16b), das mit dem zweiten Antriebskranz (19.1) in Wirkverbindung steht, über eine zweite Antriebswelle (15b), die eine zweite Antriebsachse (C') definiert, durch den zweiten Motor (17') antreibbar ist, und wobei die zwei Antriebsachsen (C, C') parallel zueinander und parallel zu der gemeinsamen Drehachse (A) der zwei Antriebsräder (18, 19; 18', 19') verlaufen.

2. Lenkgetriebe (13) nach Anspruch 1,
**dadurch gekennzeichnet, dass**
die zwei Doppelräder (18, 19) des Lenkgetriebes (13) derart in Bezug auf die Taumelscheibe (14) angeordnet sind, dass die zwei Antriebsachsen (C, C') und die gemeinsame Drehachse (A) der zwei Doppelräder (18, 19) senkrecht zu der Längsachse (B) des chirurgischen Instruments (1) verlaufen,
wobei bevorzugt die Motoren (17, 17') nebeneinander auf einer Seite in Bezug auf die Längsachse (B) angeordnet sind.

3. Lenkgetriebe (13) nach Anspruch 1,
**dadurch gekennzeichnet, dass**
das erste Antriebsrad (18, 18') als Hohl-Doppelrad (18') und
das zweite Antriebsrad (19, 19') als Hohl-Doppelrad (19')
ausgebildet sind, und
die zwei Hohl-Doppelräder (18', 19') des Lenkgetriebes (13) derart in Bezug auf die Taumelscheibe (14) angeordnet sind, dass die gemeinsame Drehachse (A) der zwei Hohl-Doppelräder (18', 19') mit der Längsachse (B) des chirurgischen Instruments (1) fluchtet und die zwei Antriebsachsen (C, C') parallel zu der Längsachse (B) des chirurgischen Instruments (1) verlaufen.

4. Lenkgetriebe (13) nach einem der Ansprüche 1 bis 3,
**dadurch gekennzeichnet, dass**
die Antriebskränze (18.1, 19.1) Zahnkränze sind, und die Ritzel (16a, 16b) Zahnräder sind, die mit den als Zahnkränzen ausgebildeten Antriebskränzen (18.1, 19.1) in Eingriff stehen.

5. Lenkgetriebe (13) nach einem der Ansprüche 1 bis 3,
**dadurch gekennzeichnet, dass**
die Antriebskränze (18.1, 19.1) und die Ritzel (16a, 16b)
- als Riemenscheiben mit einem Umfangsprofil für einen Antriebsriemen (44, 45) ausgebildet sind, wobei
ein erster Antriebsriemen (44) die Wirkverbindung des ersten Antriebskranzes (18.1) mit dem ersten Ritzel (16a) und
ein zweiter Antriebsriemen (45) die Wirkverbindung des zweiten Antriebskranzes (19.1) mit dem zweiten Ritzel (16b)
bereitstellen,
oder
- als Kettenräder für eine Antriebskette ausgebildet sind, wobei eine erste Antriebskette die Wirkverbindung des ersten Antriebskranzes (18.1) mit dem ersten Ritzel (16a) und
eine zweite Antriebskette die Wirkverbindung des zweiten Antriebskranzes (19.1) mit dem zweiten Ritzel (16b)
bereitstellen.

6. Lenkgetriebe (13) nach Anspruch 5,
**dadurch gekennzeichnet, dass**
jeder Antriebsriemen (44, 45) ein Flach-, Rund-, Keil-, Keilrippen- oder Zahnriemen ist, und das Umfangsprofil der Antriebskränze (18.1, 19.1) und der Ritzel (16a, 16b) entsprechend ein Flach-, Rund-, Keil-, Keilrippen- oder Zahnprofil ist.

7. Lenkgetriebe (13) nach einem der Ansprüche 1 bis 6,
**dadurch gekennzeichnet, dass**
die Taumelscheibe (14) mit einem dritten Zahnrad (25) gekoppelt ist, das mit den beiden Kegelradkränzen (18.2, 19.2) der beiden Doppelräder (18, 19; 18', 19') in Eingriff steht und dessen Drehachse (D) in einem rechten Winkel zu der gemeinsamen Achse (A) der Doppelräder (18, 19; 18', 19') steht,
wobei bevorzugt die Taumelscheibe (14) mit einem vierten Zahnrad (31) gekoppelt ist, das mit den beiden Kegelradkränzen (18.2, 19.2) der beiden Doppelräder (18, 19; 18', 19') gekoppelt und auf der abgewandten Seite von dem dritten Zahnrad (25) angeordnet ist.

8. Chirurgisches Instrument (1), das einen Schaft (2), eine am proximalen Ende (3) des Schaftes (2) angeordnete Betätigungseinheit (4) und ein am distalen Ende (5) des Schaftes (2) angeordnetes Instrument (7) mit einer mittels einer distalen Abwinkelungsmechanik (9) abwinkelbaren Werkzeugspitze (6) aufweist, die durch eine mittels zweier Antriebe räumlich ausrichtbare Taumelscheibe (14) steuerbar ist, **dadurch gekennzeichnet, dass**
das chirurgische Instrument (1) ein Lenkgetriebe (13) nach zumindest einem der Ansprüche 1 bis 7 aufweist, das die zwei Antriebe aufweist und dazu ausgebildet ist, die Stellwinkel der zwei Antriebe auf die räumliche Ausrichtung der Taumelscheibe (14) zu übertragen.

9. Chirurgisches Instrument (1) nach Anspruch 8,
**dadurch gekennzeichnet, dass**
die Taumelscheibe (14) zur Kopplung mit einem dritten Zahnrad (25), das mit den beiden Kegelradkränzen (18.2, 19.2) der beiden Doppelräder (18, 19; 18', 19') in Eingriff steht, um die Längsachse (B) des Schaftes (2) über einen Lagerring (32) rotierbar in einem Lenkring (30) gelagert ist, der drehfest mit dem dritten Zahnrad (25) gekoppelt ist, wobei die Taumelscheibe (14) kardanisch mit einer koaxial zu einer Längsachse (B) des Schaftes (2) verlaufenden Hauptwelle (21) gekoppelt ist.

10. Chirurgisches Instrument (1) nach Anspruch 9,
**dadurch gekennzeichnet, dass**
die Taumelscheibe (14) über zwei um 180° versetzt zueinander angeordnete Lagerstifte (27) verschwenkbar auf einer Kreuzgelenkscheibe (28) gelagert ist, wobei die Kreuzgelenkscheibe (28) über zwei um 180° versetzt zueinander angeordnete Lagerstifte (29) verschwenkbar auf der Hauptwelle (21) gelagert ist und wobei die Lagerstifte (27, 29) der Taumelscheibe (14) und der Kreuzgelenkscheibe (28) um 90 ° versetzt zueinander angeordnet sind,
oder
die kardanische Lagerung durch zwei sich längs erstreckende, diametral in der Hauptwelle (21) vorliegende Führungsnuten und zwei diametral und radial nach innen weisend an der Taumelscheibe (14) angeordnete Stifte bereitgestellt wird, wobei jeder Stift in eine der Führungsnuten eingreift, sodass ein Drehwinkel der Hauptwelle (21) auf die Taumelscheibe (14) übertragbar ist.

11. Chirurgisches Instrument (1) nach zumindest einem der Ansprüche 8 bis 10, **dadurch gekennzeichnet, dass**
in Längsrichtung (B) des Schaftes (2) Lenkdrähte (12) verlaufen, die mit der Taumelscheibe (14) des Lenkgetriebes (13) verbunden sind.

12. Chirurgisches Instrument (1) nach Anspruch 11,
**dadurch gekennzeichnet, dass**
das vierte Zahnrad (31) mit der Taumelscheibe (14) über einen Lagerring (42) mit dem Lenkring (30) gekoppelt ist, wobei das vierte Zahnrad (31) gegenüber dem dritten Zahnrad (25) frei drehbar ist.

13. Chirurgisches Instrument (1) nach zumindest einem der Ansprüche 11 oder 12, **dadurch gekennzeichnet, dass**
ein Betätigungselement (8) axial verschiebbar in dem Schaft (2) gelagert ist und proximalseitig mit der Betätigungseinheit (4) in Wirkverbindung steht, und dass die distale Abwinkelungsmechanik (9) der abwinkelbaren Werkzeugspitze (6) aus an dem distalen Ende (5) des Schaftes (2) angeordneten Schwenkgliedern (11) besteht, die über in Längsrichtung (B) des Schaftes (2) verlaufende Lenkdrähte (12) mit dem Lenkgetriebe (13) verbunden sind.

14. Chirurgisches Instrument (1) nach zumindest einem der Ansprüche 11 bis 13, **dadurch gekennzeichnet, dass**
ein radialer Abstand der Lenkdrähte (12) von der Längsachse (B) des Schaftes (2) an der Taumelscheibe (14) größer ist als am proximalen Ende (3) des Schaftes (2), aus dem die Lenkdrähte (12) austreten, wobei
- die Lenkdrähte (12) sich von dem proximalen Ende (3) des Schaftes (2) direkt zu der Taumelscheibe (14) erstrecken, wobei die Lenkdrähte (12) unter einem von 90° abweichenden Winkel zur Taumelscheibe (14) verlaufen,
oder
- distalseitig vor der Taumelscheibe (14) eine Fächerscheibe (22) auf der Hauptwelle (21) angeordnet ist, die den radialen Abstand der Lenkdrähte (12) von der Längsachse (B) des Schaftes (2) vergrößert, sodass die Lenkdrähte (12) zwischen der Fächerscheibe (22) und der Taumelscheibe (14) parallel zueinander verlaufen und in Bezug auf eine Scheibenfläche der Taumelscheibe (14) einen Winkel von 90 ° bilden.

## Claims

1. A steering gear (13) for a surgical instrument (1) which can be arranged at the proximal end (3) of a shaft (2), which defines a longitudinal axis (B) and has a bending mechanism (9) at the distal end (5),
wherein the steering gear (13) has two motorised drives and a swash plate (14), and is designed to spatially align the swash plate (14) via the adjustment angles of the two drives, which swash plate is designed to control the distal bending mechanism (9) of the surgical instrument (1),
wherein
the first drive has a first drive wheel (18, 18') which is driven by a first motor (17) and is designed as a double wheel (18, 18') with a first bevel gear rim (18.2) and a first drive rim (18.1), and the second drive has a second drive wheel (19, 19') which is driven by a second motor (17') and is designed as a double wheel (19, 19') with a second bevel gear rim (19.2) and a second drive rim (19.1),
wherein
the swash plate (14) is arranged between the two drive wheels (18, 19; 18', 19'), which have a common axis of rotation (A), and the bevel gear rims (18.2, 19.2) face one another, and
wherein
the drive rims (18.1, 19.1) of both double wheels (18, 19; 18', 19') are each operatively connected to a pinion (16a, 16b),
wherein a first pinion (16a), which is operatively connected to the first drive rim (18.1), can be driven by the first motor (17) via a first drive shaft (15a), which defines a first drive axis (C), and a second pinion (16b), which is operatively connected to the second drive rim (19.1), can be driven by the second motor (17') via a second drive shaft (15b), which defines a second drive axis (C'), and
wherein the two drive axes (C, C') run parallel to each other and parallel to the common axis of rotation (A) of the two drive wheels (18, 19; 18', 19').

2. The steering gear (13) according to claim 1,
**characterised in that**
the two double wheels (18, 19) of the steering gear (13) are arranged in relation to the swash plate (14) in such manner that the two drive axes (C, C') and the common axis of rotation (A) of the two double wheels (18, 19) run perpendicular to the longitudinal axis (B) of the surgical instrument (1),
wherein the motors (17, 17') are preferably arranged next to each other on one side in relation to the longitudinal axis (B).

3. The steering gear (13) according to claim 1,
**characterised in that**
the first drive wheel (18, 18') is designed as a hollow double wheel (18') and
the second drive wheel (19, 19') is designed as a hollow double wheel (19'), and
the two hollow double wheels (18', 19') of the steering gear (13) are arranged in relation to the swash plate (14) in such manner that the common axis of rotation (A) of the two hollow double wheels (18', 19') is aligned with the longitudinal axis (B) of the surgical instrument (1) and the two drive axes (C, C') run parallel to the longitudinal axis (B) of the surgical instrument (1).

4. The steering gear (13) according to one of claims 1 to 3,
**characterised in that**
the drive rims (18.1, 19.1) are gear rims, and the pinions (16a, 16b) are gear wheels which engage with the drive rims (18.1, 19.1) designed as gear rims.

5. The steering gear (13) according to one of claims 1 to 3,
**characterised in that**
the drive rims (18.1, 19.1) and the pinions (16a, 16b)
- are designed as belt pulleys with a circumferential profile for a drive belt (44, 45), wherein
a first drive belt (44) provides the operative connection between the first drive rim (18.1) and the first pinion (16a), and
a second drive belt (45) provides the operative connection between the second drive rim (19.1) and the second pinion (16b),
or
- are designed as sprockets for a drive chain, wherein
a first drive chain provides the operative connection between the first drive rim (18.1) and the first pinion (16a), and
a second drive chain provides the operative connection between the second drive rim (19.1) and the second pinion (16b).

6. The steering gear (13) according to claim 5,
**characterised in that**
each drive belt (44, 45) is a flat, round, V-, V-ribbed or toothed belt, and the circumferential profile of the drive rims (18.1, 19.1) and the pinions (16a, 16b) is correspondingly a flat, round, V-, V-ribbed or toothed profile.

7. The steering gear (13) according to one of claims 1 to 6,
**characterised in that**
the swash plate (14) is coupled to a third gear wheel (25), which is in engagement with the two bevel gear rims (18.2, 19.2) of the two double wheels (18, 19; 18', 19') and whose axis of rotation (D) is at a right angle to the common axis of rotation (A) of the driven double wheels (18, 19; 18', 19'),
wherein the swash plate (14) is preferably coupled to a fourth gear wheel (31) which is coupled to the two bevel gear rims (18.2, 19.2) of the two double wheels (18, 19; 18', 19') and is arranged on the side facing away from the third gear wheel (25).

8. A surgical instrument (1) having a shaft (2), an actuation unit (4) arranged at the proximal end (3) of the shaft (2) and an instrument (7) arranged at the distal end (5) of the shaft (2) with a tool tip (6) which can be angled by means of a distal bending mechanism (9) and which can be controlled by a swash plate (14) which can be spatially aligned by means of two drives,
**characterised in that**
the surgical instrument (1) has a steering gear (13) according to at least one of claims 1 to 7, which has the two drives and is designed
to transfer the adjustment angles of the two drives to the spatial orientation of the swash plate (14).

9. The surgical instrument (1) according to claim 8,
**characterised in that**
the swash plate (14), for coupling with a third gear wheel (25), which is in engagement with the two bevel gear rims (18.2, 19.2) of the two double wheels (18, 19; 18', 19'), is mounted rotatably about the longitudinal axis (B) of the shaft (2) via a bearing ring (32) in a steering ring (30), which is coupled to the third gear wheel (25) in a rotationally-fixed manner, wherein the swash plate (14) is coupled in a cardanic manner to a main shaft (21) running coaxially to a longitudinal axis (B) of the shaft (2).

10. The surgical instrument (1) according to claim 9,
**characterised in that**
the swash plate (14) is pivotably mounted on a universal joint disc (28) via two bearing pins (27) arranged offset by 180° relative to one another, wherein the universal joint disc (28) is pivotably mounted on the main shaft (21) via two bearing pins (29) arranged offset by 180° relative to one another and wherein the bearing pins (27, 29) of the swash plate (14) and the universal joint disc (28) are arranged offset by 90° relative to one another,
or
the cardanic mounting is provided by two longitudinally extending guide grooves located diametrically in the main shaft (21) and two pins arranged diametrically and pointing radially inwards on the swash plate (14),
wherein each pin engages in one of the guide grooves such that an angle of rotation of the main shaft (21) can be transmitted to the swash plate (14).

11. The surgical instrument (1) according to at least one of claims 8 to 10,
**characterised in that**
steering wires (12) run in the longitudinal direction (B) of the shaft (2) and are connected to the swash plate (14) of the steering gear (13).

12. The surgical instrument (1) according to claim 11,
**characterised in that**
the fourth gear wheel (31) is coupled to the swash plate (14) via a bearing ring (42) with the steering ring (30), wherein the fourth gear wheel (31) is freely rotatable relative to the third gear wheel (25).

13. The surgical instrument (1) according to at least one of claims 11 or 12,
**characterised in that**
an actuating element (8) is mounted so as to be axially displaceable in the shaft (2) and is operatively connected to the actuation unit (4) at the proximal side, and **in that** the distal bending mechanism (9) of the bendable tool tip (6) consists of swivel members (11) which are arranged at the distal end (5) of the shaft (2) and which are connected to the steering gear (13) via steering wires (12) running in the longitudinal direction (B) of the shaft (2).

14. The surgical instrument (1) according to at least one of claims 11 to 13,
**characterised in that**
a radial distance of the steering wires (12) from the longitudinal axis (B) of the shaft (2) is greater at the swash plate (14) than at the proximal end (3) of the shaft (2) from which the steering wires (12) emerge, wherein
- the steering wires (12) extend from the proximal end (3) of the shaft (2) directly to the swash plate (14), wherein the steering wires (12) run at an angle to the swash plate (14) that deviates from 90°,
or
- on the distal side in front of the swash plate (14) is arranged a serrated lock washer (22) on the main shaft (21), which increases the radial distance of the steering wires (12) from the longitudinal axis (B) of the shaft (2) such that the steering wires (12) run parallel to each other between the serrated lock washer (22) and the swash plate (14) and form an angle of 90° in relation to a plate surface of the swash plate (14).

## Revendications

1. Appareil de direction (13) pour un instrument chirurgical (1) qui peut être disposé à l'extrémité proximale (3) d'un arbre (2), qui définit un axe longitudinal (B) et a un mécanisme de flexion (9) au niveau de l'extrémité distale (5),
dans lequel le mécanisme de direction (13) a deux entraînements motorisés et un plateau oscillant (14) et est conçu de manière à orienter dans l'espace un plateau oscillant (14) par l'intermédiaire des angles de réglage des deux entraînements, ce plateau étant conçu de manière à commander un mécanisme de flexion distal (9) de l'instrument chirurgical (1),
dans lequel
le premier entraînement a une première roue d'entraînement (18, 18') entraînée par un premier moteur (17) et conçue comme une roue double (18, 18') avec une première couronne conique (18.2) et un premier bord d'entraînement (18.1), et le deuxième entraînement a une deuxième roue d'entraînement (19, 19') entraînée par un deuxième moteur (17') et conçue comme une roue double (19, 19') avec une deuxième couronne conique (19.2) et un deuxième bord d'entraînement (19.1),
dans lequel
le plateau oscillant (14) est disposé entre les deux roues d'entraînement (18, 19 ; 18', 19'), qui ont un axe de rotation commun (A), et les couronnes coniques (18.2, 19.2) se font face, et
dans lequel
les bords d'entraînement (18.1, 19.1) des deux roues doubles (18, 19 ; 18', 19') sont chacun reliés de manière opérationnelle à un pignon (16a, 16b),
dans lequel un premier pignon (16a), qui est relié de manière opérationnelle au premier bord d'entraînement (18.1), peut être entraîné par le premier moteur (17) par l'intermédiaire d'un premier arbre d'entraînement (15a), qui définit un premier axe d'entraînement (C), et un deuxième pignon (16b), qui est relié de manière opérationnelle au deuxième bord d'entraînement (19.1), peut être entraîné par le deuxième moteur (17') par l'intermédiaire d'un deuxième arbre d'entraînement (15b), qui définit un deuxième axe d'entraînement (C), et
dans lequel les deux axes d'entraînement (C, C') s'étendent parallèles entre elles et parallèles à l'axe de rotation commun (A) des deux roues d'entraînement (18, 19 ; 18', 19').

2. Appareil de direction (13) selon la revendication 1,
**caractérisé en ce que**
les deux roues doubles (18, 19) de l'appareil de direction (13) sont disposées par rapport au plateau oscillant (14) de manière à ce que les deux axes d'entraînement (C, C') et l'axe de rotation commun (A) des deux roues doubles (18, 19) s'étend perpendiculaire à l'axe longitudinal (B) de l'instrument chirurgical (1),
dans lequel les moteurs (17, 17') sont de préférence disposés l'un à côté de l'autre sur un côté par rapport à l'axe longitudinal (B).

3. Appareil de direction (13) selon la revendication 1,
**caractérisé en ce que**
la première roue d'entraînement (18, 18') est conçue comme une roue double creuse (18') et
la deuxième roue d'entraînement (19, 19') est conçue comme une roue double creuse (19'), et
les deux roues doubles creuses (18', 19') de l'appareil de direction (13) sont disposées par rapport au plateau oscillant (14) de manière à ce que l'axe de rotation commun (A) des deux roues doubles creuses (18', 19') soit aligné avec l'axe longitudinal (B) de l'instrument chirurgical (1) et les deux axes d'entraînement (C, C') s'étendent parallèles à l'axe longitudinal (B) de l'instrument chirurgical (1).

4. Appareil de direction (13) selon l'une des revendications 1 à 3,
**caractérisé en ce que**
les bords d'entraînement (18.1, 19.1) sont des bords de couronnes, et les pignons (16a, 16b) sont des roues dentées qui sont en prise avec les bords d'entraînement (18.1, 19.1) conçues comme des bords de couronnes.

5. Appareil de direction (13) selon l'une des revendications 1 à 3,
**caractérisé en ce que**
les bords d'entraînement (18.1, 19.1) et les pignons (16a, 16b)
- sont conçus comme des poulies à courroie avec un profil circonférentiel pour une courroie d'entraînement (44, 45), dans lequel
une première courroie d'entraînement (44) fournit la liaison opérationnelle entre le premier bord d'entraînement (18.1) et le premier pignon (16a), et
une deuxième courroie d'entraînement (45) fournit la liaison opérationnelle entre le deuxième bord d'entraînement (19.1) et le deuxième pignon (16b),
ou
- sont conçus comme des pignons pour une chaîne d'entraînement, dans lequel
une première chaîne d'entraînement fournit la liaison opérationnelle entre le premier bord d'entraînement (18.1) et le premier pignon (16a), et
une deuxième chaîne d'entraînement fournit la liaison opérationnelle entre le deuxième bord d'entraînement (19.1) et le deuxième pignon (16b).

6. Appareil de direction (13) selon la revendication 5,
**caractérisé en ce que**
chaque courroie d'entraînement (44, 45) est une courroie plate, ronde, en V, nervurée en V ou dentée, et le profil circonférentiel des bords d'entraînement (18.1, 19.1) et des pignons (16a, 16b) est respectivement un profil plat, rond, en V, nervurée en V ou denté.

7. Appareil de direction (13) selon l'une des revendications 1 à 6,
**caractérisé en ce que**
le plateau oscillant (14) est couplé à une troisième roue dentée (25), qui est en prise avec les deux couronnes coniques (18.2, 19.2) des deux roues doubles (18, 19 ; 18', 19') et dont l'axe de rotation (D) est à angle droit par rapport à l'axe de rotation commun (A) des roues doubles entraînées (18, 19 ; 18', 19'),
dans lequel le plateau oscillant (14) est de préférence couplé à une quatrième roue dentée (31) qui est couplée aux deux couronnes coniques (18.2, 19.2) des deux roues doubles (18, 19 ; 18', 19') et est disposé sur le côté opposé à la troisième roue dentée (25).

8. Instrument chirurgical (1) ayant un arbre (2), une unité d'actionnement (4) disposée à l'extrémité proximale (3) de l'arbre (2) et un instrument (7) disposé à l'extrémité distale (5) de l'arbre (2) avec une pointe d'outil (6) qui peut être inclinée au moyen d'un mécanisme de flexion distale (9) et qui peut être contrôlée par un plateau oscillant (14) qui peut être aligné dans l'espace au moyen de deux entraînements,
**caractérisé en ce que**
l'instrument chirurgical (1) a un appareil de direction (13) selon au moins l'une des revendications 1 à 7, qui a les deux entraînements et est conçu
pour transférer les angles de réglage des deux entraînements à l'orientation spatiale du plateau oscillant (14).

9. Instrument chirurgical (1) selon la revendication 8,
**caractérisé en ce que**
le plateau oscillant (14), pour l'accouplement avec une troisième roue dentée (25), qui est en prise avec les deux couronnes coniques (18.2, 19.2) des deux roues doubles (18, 19 ; 18', 19'), est monté rotatif autour de l'axe longitudinal (B) de l'arbre (2) par l'intermédiaire d'une bague de roulement (32) dans une bague de direction (30), qui est couplée à la troisième roue dentée (25) de manière fixe en rotation, dans lequel le plateau oscillant (14) est couplé de manière cardanique à un arbre principal (21) coaxial à l'axe longitudinal (B) de l'arbre (2).

10. Instrument chirurgical (1) selon la revendication 9,
**caractérisé en ce que**
le plateau oscillant (14) est monté pivotant sur un disque de joint universel (28) par l'intermédiaire de deux goupilles d'appui (27) décalés de 180° l'un par rapport à l'autre, dans lequel le disque de joint universel (28) est monté pivotant sur l'arbre principal (21) par l'intermédiaire de deux goupilles d'appui (29) décalés de 180° l'une par rapport à l'autre et dans lequel les goupilles d'appui (27, 29) du plateau oscillant (14) et du disque de joint universel (28) sont décalés de 90° l'une par rapport à l'autre,
ou
le montage cardanique est fourni par deux rainures de guidage s'étendant longitudinalement et situées diamétralement dans l'arbre principal (21) et deux goupilles disposées diamétralement et orientées radialement vers l'intérieur sur le plateau oscillant (14),
dans lequel chaque goupille s'engage dans l'une des rainures de guidage de sorte qu'un angle de rotation de l'arbre principal (21) puisse être transmis au plateau oscillant (14).

11. Instrument chirurgical (1) selon au moins l'une des revendications 8 à 10,
**caractérisé en ce que**
les fils de direction (12) s'étendent dans la direction longitudinale (B) de l'arbre (2) et sont reliés au plateau oscillant (14) de l'appareil de direction (13).

12. Instrument chirurgical (1) selon la revendication 11,
**caractérisé en ce que**
la quatrième roue dentée (31) est couplée au plateau oscillant (14) par l'intermédiaire d'une bague de roulement (42) avec la bague de direction (30), dans lequel la quatrième roue dentée (31) peut tourner librement par rapport à la troisième roue dentée (25).

13. Instrument chirurgical (1) selon au moins l'une des revendications 11 ou 12,
**caractérisé en ce que**
un élément d'actionnement (8) est monté de manière à pouvoir être déplacé axialement dans l'arbre (2) et est relié de manière opérationnelle à l'unité d'actionnement (4) sur le côté proximal, et **en ce que** le mécanisme de flexion distale (9) de la pointe de l'outil pliable (6) constitue des éléments pivotants (11) qui sont disposés à l'extrémité distale (5) de l'arbre (2) et qui sont reliés à l'appareil de direction (13) par des fils de direction (12) s'étendant dans la direction longitudinale (B) de l'arbre (2).

14. Instrument chirurgical (1) selon au moins l'une des revendications 11 à 13,
**caractérisé en ce que**
une distance radiale des fils de direction (12) depuis l'axe longitudinal (B) de l'arbre (2) est plus grande au niveau du plateau oscillant (14) qu'à l'extrémité proximale (3) de l'arbre (2) d'où sortent les fils de direction (12), dans lequel
- les fils de direction (12) s'étendent de l'extrémité proximale (3) de l'arbre (2) directement au plateau oscillant (14), dans lequel les fils de direction (12) forment avec le plateau oscillant (14) un angle qui diffère de 90°,
ou
- sur le côté distal, devant le plateau oscillant (14), une rondelle éventail (22) est disposée sur l'arbre principal (21), qui augmente la distance radiale des fils de direction (12) depuis l'axe longitudinal (B) de l'arbre (2) de telle sorte que les fils de direction (12) soient parallèles entre la rondelle éventail (22) et le plateau oscillant (14) et forment un angle de 90° par rapport à la surface de plateau du plateau oscillant (14).
